# EUROPEAN PATENT APPLICATION

(11) **EP 4 541 284 A1**
(43) Date of publication of application: **23.04.2025**
(21) Application number: 23823581.6
(22) Date of filing: 15.05.2023
(51) Int. Cl.: A61B 6/00

(54) **MEDICAL IMAGE PROCESSING DEVICE AND MEDICAL IMAGE PROCESSING METHOD**

(30) Priority: 15.06.2022 JP 2022096348
(71) Applicant: Renaissance of Technology Corporation, Hamamatsu-shi, Shizuoka 431-3126 (JP)
(72) Inventor: KOSUGI, Takashi, Hamamatsu-shi, Shizuoka 431-3126 (JP); KOSUGI, Takafumi, Hamamatsu-shi, Shizuoka 431-3126 (JP)
(74) Representative: Vossius & Partner Patentanwälte Rechtsanwälte mbB
(86) International application number: PCT/JP2023/018088
(87) International publication number: WO 2023/243280

(57) **Abstract**

In a medical image processing apparatus (300) includes a display unit (380) for a surgeon (20) to view an operation on a blood vessel of a subject using a medical device (100), a display processing unit (370) generates a three-dimensional blood vessel image (371) based on a transparency (305) of the blood vessel estimated by a transparency estimation unit (350), generates a three-dimensional medical device image (372) based on three-dimensional device shape point cloud information (307) of a point cloud belonging to a group determined to be a real image by a real/virtual image determination unit (360), and performs processing of displaying, on the display unit (380), a three-dimensional medical image (309) generated by superimposing the three-dimensional blood vessel image (371) and the three-dimensional medical device image (372).

## Description

### TECHNICAL FIELD

The present invention relates to a medical image processing apparatus including a display unit for a surgeon to view an operation on a blood vessel of a subject using a medical device, and a medical image processing method using the medical image processing apparatus.

### BACKGROUND ART

For a surgeon to perform an operation on a blood vessel of a subject using a medical device, an operation has been performed in which an X-ray fluoroscopic image, which is a type of medical image obtained by X-ray fluoroscopy of a region of the subject including the blood vessel and the medical device, is displayed as a video on a display unit, allowing the surgeon to view the X-ray fluoroscopic image displayed on the display unit during the operation. For example, Patent Literature 1 describes a technology in which, in performing an operation with a medical device (specifically, a stent) placed in a blood vessel, a region of the subject including the blood vessel and the medical device is captured by X-ray fluoroscopy to display the resulting X-ray fluoroscopic image as a video on a display unit.

### CITATION LIST

### PATENT LITERATURE

Patent Literature 1: Japanese Laid-open Patent Publication No. 2018-114203

### SUMMARY OF INVENTION

### TECHNICAL PROBLEM

Generally, an X-ray fluoroscopic images is a two-dimensional image, and with the technology described in Patent Literature 1, therefore, the surgeon proceeds with operation while viewing the two-dimensional image to recognize a three-dimensional positional relationship between the blood vessel and the medical device. In this case, since two-dimensional information has a lack of information compared to three-dimensional information, the technology described in Patent Literature 1 is insufficient from the viewpoint of enabling the surgeon to safely proceed with vascular operation.

The present invention has been made in consideration of such a problem, and an object thereof is to provide a mechanism that, for a surgeon to perform an operation on a blood vessel of a subject using a medical device while viewing a medical image, makes it possible to perform the operation safely by more easily recognizing a three-dimensional positional relationship between the blood vessel and the medical device.

### SOLUTION TO PROBLEM

A medical image processing apparatus of the present invention includes a display unit for a surgeon to view an operation on a blood vessel of a subject using a medical device, the medical image processing apparatus including: a feature amount calculation unit configured to calculate three-dimensional feature amounts of the blood vessel and the medical device based on a first-plane two-dimensional image, first-plane imaging information, a second-plane two-dimensional image, and second-plane imaging information, the first-plane two-dimensional image including a first-plane X-ray fluoroscopic image of the subject including the blood vessel into which the medical device is inserted, captured in a direction of a first-plane by X-ray fluoroscopy, and including a first-plane roadmap image corresponding to the first-plane X-ray fluoroscopic image, the first-plane imaging information being related to X-ray fluoroscopy in the direction of the first plane, the second-plane two-dimensional image including a second-plane X-ray fluoroscopic image of the subject including the blood vessel into which the medical device is inserted, captured in a direction of a second-plane different from the direction of the first plane by X-ray fluoroscopy, and including a second-plane roadmap image corresponding to the second-plane X-ray fluoroscopic image, the second-plane imaging information being related to X-ray fluoroscopy in the direction of the second plane; a two-dimensional device shape image generation unit configured to generate a first-plane two-dimensional device shape image representing a shape of the medical device in the direction of the first plane based on the first-plane two-dimensional image, and generate a second-plane two-dimensional device shape image representing a shape of the medical device in the direction of the second plane based on the second-plane two-dimensional image; a three-dimensional device shape point cloud information calculation unit configured to calculate three-dimensional device shape point cloud information that is point cloud information of a three-dimensional shape of the medical device, based on the first-plane two-dimensional device shape image, the second-plane two-dimensional device shape image, the first-plane imaging information, and the second-plane imaging information; a transparency estimation unit configured to estimate a transparency of the blood vessel based on the three-dimensional feature amounts calculated by the feature amount calculation unit and subject lattice point cloud information that is information on a plurality of lattice points set for the subject, and estimate a transparency of the medical device based on the three-dimensional feature amounts calculated by the feature amount calculation unit and the three-dimensional device shape point cloud information; a real/virtual image determination unit configured to determine, based on the transparency of the medical device estimated by the transparency estimation unit, whether the three-dimensional device shape point cloud information indicates a real image or a virtual image for each of a plurality of groups into which a nearby region including a point cloud in the three-dimensional device shape point cloud information is divided; and a display processing unit configured to generate a three-dimensional blood vessel image based on the transparency of the blood vessel estimated by the transparency estimation unit, generate a three-dimensional medical device image based on the three-dimensional device shape point cloud information of the point cloud belonging to the group determined to be a real image by the real/virtual image determination unit, and perform processing of displaying on the display unit a three-dimensional medical image generated by superimposing the three-dimensional blood vessel image and the three-dimensional medical device image.
Further, the present invention includes a medical image processing method using the above-described medical image processing apparatus.

### ADVANTAGEOUS EFFECTS OF INVENTION

According to the present invention, for a surgeon to perform an operation on a blood vessel of a subject using a medical device while viewing a medical image, it is possible to perform the operation safely.

### BRIEF DESCRIPTION OF DRAWINGS

[Fig. 1] Fig. 1 is a diagram illustrating an example of a schematic configuration of a medical image processing system according to an embodiment of the present invention.
[Fig. 2A] Fig. 2A illustrates the embodiment of the present invention and is an image view illustrating a specific example of a first-plane X-ray fluoroscopic image in Fig. 1.
[Fig. 2B] Fig. 2B illustrates the embodiment of the present invention and is an image view illustrating a specific example of a first-plane roadmap image in Fig. 1.
[Fig. 3A] Fig. 3A illustrates the embodiment of the present invention and illustrates specific examples of first-plane imaging information and second-plane imaging information in Fig. 1.
[Fig. 3B] Fig. 3B is a diagram for explaining various types of information included in the first-plane imaging information and the second-plane imaging information illustrated in Fig. 3A.
[Fig. 3C] Fig. 3C is a diagram for explaining various types of information included in the first-plane imaging information and the second-plane imaging information illustrated in Fig. 3A.
[Fig. 3D] Fig. 3D is a diagram for explaining various types of information included in the first-plane imaging information and the second-plane imaging information illustrated in Fig. 3A.
[Fig. 3E] Fig. 3E is a diagram for explaining various types of information included in the first-plane imaging information and the second-plane imaging information illustrated in Fig. 3A.
[Fig. 3F] Fig. 3F is a diagram for explaining various types of information included in the first-plane imaging information and the second-plane imaging information illustrated in Fig. 3A.
[Fig. 3G] Fig. 3G is a diagram for explaining various types of information included in the first-plane imaging information and the second-plane imaging information illustrated in Fig. 3A.
[Fig. 4] Fig. 4 illustrates the embodiment of the present invention and is a diagram illustrating a specific example of subject lattice point cloud information in Fig. 1.
[Fig. 5] Fig. 5 illustrates the embodiment of the present invention and is a diagram illustrating an example of processing performed by a feature amount calculation unit in Fig. 1.
[Fig. 6] Fig. 6 illustrates the embodiment of the present invention and is a diagram illustrating an example of processing performed by a two-dimensional device shape image generation unit in Fig. 1.
[Fig. 7] Fig. 7 illustrates the embodiment of the present invention and is a diagram illustrating an example of processing performed by a three-dimensional device shape point cloud information calculation unit in Fig. 1.
[Fig. 8] Fig. 8 illustrates the embodiment of the present invention and is a diagram illustrating an example of processing performed by a transparency estimation unit in Fig. 1.
[Fig. 9] Fig. 9 illustrates the embodiment of the present invention and is a diagram illustrating an example of processing performed by a real/virtual image determination unit in Fig. 1.
[Fig. 10] Fig. 10 illustrates the embodiment of the present invention and is a diagram illustrating an example of processing performed by a display processing unit in Fig. 1.
[Fig. 11] Fig. 11 is a flowchart illustrating an example of a processing procedure of a medical image processing method by the medical image processing apparatus according to the embodiment of the present invention.

### DESCRIPTION OF EMBODIMENTS

An embodiment of the present invention will be described below with reference to the drawings. In the embodiment of the present invention described below, an example will be described in which the head of a patient is used as a subject to be subjected to X-ray fluoroscopy.

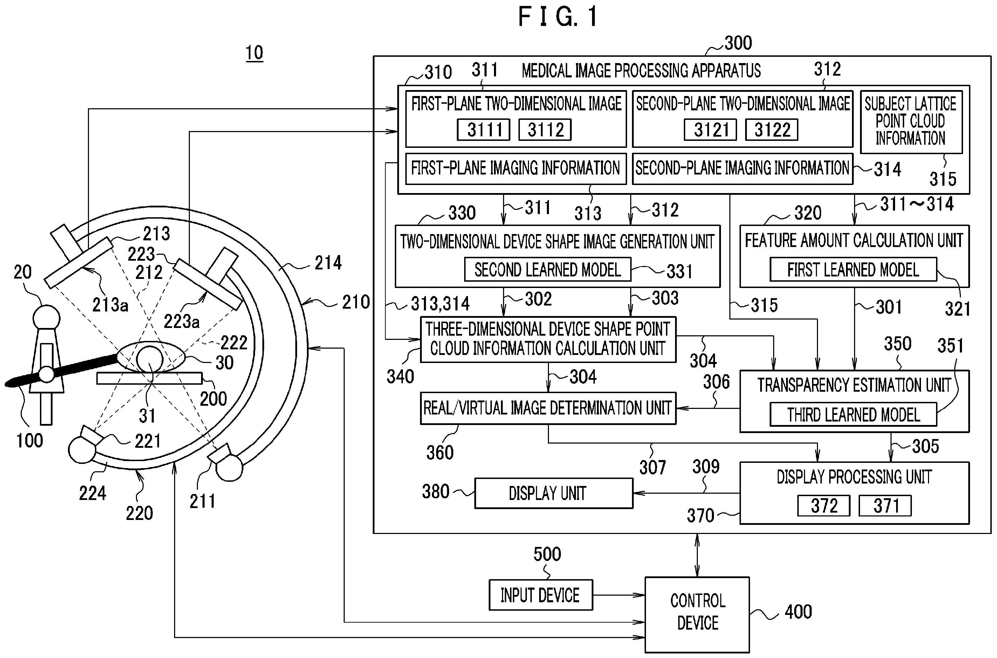

Fig. 1 is a diagram illustrating an example of a schematic configuration of a medical image processing system 10 according to the embodiment of the present invention. As illustrated in Fig. 1, the medical image processing system 10 is configured to include a medical device 100, a tabletop 200, a first X-ray imaging apparatus 210, a second X-ray imaging apparatus 220, a medical image processing apparatus 300, a control device 400, and an input device 500.

The medical device 100 is a medical device used by a surgeon 20 to perform an operation on a blood vessel in a head 31 of a patient 30 who is a subject. In the present embodiment, the medical device 100 is, for example, a catheter instrument (including various types of catheters and coils) for performing coil embolization on an aneurysm of a cerebral artery inside the head 31 of the patient 30.

The tabletop 200 is a member on which the patient 30 rests, and is made of a material that transmits X-rays.

The first X-ray imaging apparatus 210 includes an X-ray generation unit 211, an X-ray detection unit 213, and a C-arm 214. This first X-ray imaging apparatus 210 is a device that uses X-rays 212 to capture an image of an inside of the head 31 of the patient 30 (a region including a blood vessel into which the medical device 100 is inserted) on an X-ray detection surface 213a of the X-ray detection unit 213 that is in the direction of a first plane. The X-ray generation unit 211 generates the X-rays 212 toward the head 31 of the patient 30 and the X-ray detection unit 213 under the control of the control device 400. The X-ray detection unit 213 detects the X-rays 212 that have passed through the head 31 of the patient 30 as an image signal under the control of the control device 400. The C-arm 214 is a support member for fixing the X-ray generation unit 211 to one end and the X-ray detection unit 213 to the other end, and for positioning the X-ray generation unit 211 and the X-ray detection unit 213 to face each other and to interpose the head 31 of the patient 30, who is a subject, between them. The C-arm 214 is configured to be movable, for example, under the control of the control device 400.

The second X-ray imaging apparatus 220 is configured to include an X-ray generation unit 221, an X-ray detection unit 223, and a C-arm 224. This second X-ray imaging apparatus 220 is a device that uses X-rays 222 to capture an image of an inside of the head 31 of the patient 30 (a region including a blood vessel into which the medical device 100 is inserted) on an X-ray detection surface 223a of the X-ray detection unit 223 that is in the direction of a second plane. The X-ray generation unit 221 generates the X-rays 222 toward the head 31 of the patient 30 and the X-ray detection unit 223 under the control of the control device 400. The X-ray detection unit 223 detects the X-rays 222 that have passed through the head 31 of the patient 30 as an image signal under the control of the control device 400. The C-arm 224 is a support member for fixing the X-ray generation unit 221 to one end and the X-ray detection unit 223 to the other end, and for positioning the X-ray generation unit 221 and the X-ray detection unit 223 to face each other and to interpose the head 31 of the patient 30, who is a subject, between them. The C-arm 224 is configured to be movable, for example, under the control of the control device 400.

The medical image processing apparatus 300 performs processing of generating a three-dimensional medical image to be viewed by the surgeon 20 to perform operation on a blood vessel in the head 31 of the patient 30, who is a subject, using the medical device 100. Each internal component of this medical image processing apparatus 300 will be described later.

The control device 400 generally controls the operation of the medical image processing system 10 and performs various types of processes, based on information input from the input device 500, for example.

The input device 500 receives various types of information to be input to the control device 400.

Next, each internal component of the medical image processing apparatus 300 will be described. As illustrated in Fig. 1, the medical image processing apparatus 300 is configured to include an image/information acquisition unit 310, a feature amount calculation unit 320, a two-dimensional device shape image generation unit 330, a three-dimensional device shape point cloud information calculation unit 340, a transparency estimation unit 350, a real/virtual image determination unit 360, a display processing unit 370, and a display unit 380.

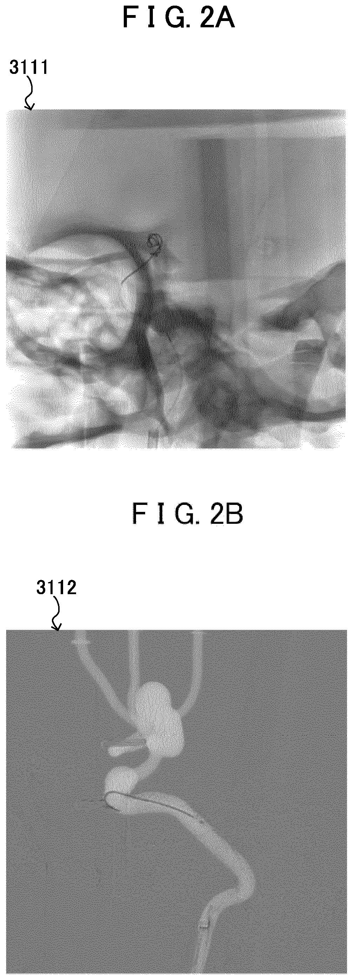

The image/information acquisition unit 310 acquires, from the X-ray detection unit 213 of the first X-ray imaging apparatus 210, a first-plane two-dimensional image 311 including: a first-plane X-ray fluoroscopic image 3111 of the head 31 of the patient 30, who is a subject, captured by X-ray fluoroscopy on the X-ray detection surface 213a of the X-ray detection unit 213, which is the direction of the first plane; and a first-plane roadmap image 3112 corresponding to the first-plane X-ray fluoroscopic image 3111. Figs. 2A and 2B illustrate the embodiment of the present invention and are image views illustrating specific examples of the first-plane X-ray fluoroscopic image 3111 and the first-plane roadmap image 3112 in Fig. 1, respectively. The first-plane X-ray fluoroscopic image 3111 illustrated in Fig. 2A is raw data of an X-ray fluoroscopic image of the head 31 of the patient 30, who is a subject, captured by X-ray fluoroscopy on the X-ray detection surface 213a of the X-ray detection unit 213, which is in the direction of the first plane. In this first-plane X-ray fluoroscopic image 3111 illustrated in Fig. 2A, a region is depicted, including the skull of the head 31 of the patient 30 and the blood vessel into which the medical device 100 is inserted. The first-plane roadmap image 3112 illustrated in Fig. 2B is an image obtained by superimposing an angiographic image captured immediately before in the direction of the first plane and the first-plane X-ray fluoroscopic image 3111. In the present embodiment, the first-plane roadmap image 3112 is an image in which the blood vessel is stained with a contrast agent, or an image in which the blood vessel is highlighted in white as illustrated in Fig. 2B.

Now, return to Fig. 1 again for explanation. The image/information acquisition unit 310 acquires, from the X-ray detection unit 223 of the second X-ray imaging apparatus 220, a second-plane two-dimensional image 312 including: a second-plane X-ray fluoroscopic image 3121 of the head 31 of the patient 30, who is a subject, captured by X-ray fluoroscopy on the X-ray detection surface 223a of the X-ray detection unit 223, which is the direction of the second plane; and a second-plane roadmap image 3122 corresponding to the second-plane X-ray fluoroscopic image 3121. The second-plane X-ray fluoroscopic image 3121 is raw data of an X-ray fluoroscopic image captured by X-ray fluoroscopy in the direction of the second plane different from the direction of the first plane in the first-plane X-ray fluoroscopic image 3111 illustrated in Fig. 2A. Further, the second-plane roadmap image 3122 is an image obtained by superimposing an angiographic image captured immediately before in the direction of the second plane and the second-plane X-ray fluoroscopic image 3121. In the present embodiment, the second-plane roadmap image 3122 is an image in which the blood vessel is stained with a contrast agent, or an image in which the blood vessel is highlighted in white like the first-plane roadmap image 3112 illustrated in Fig. 2B.

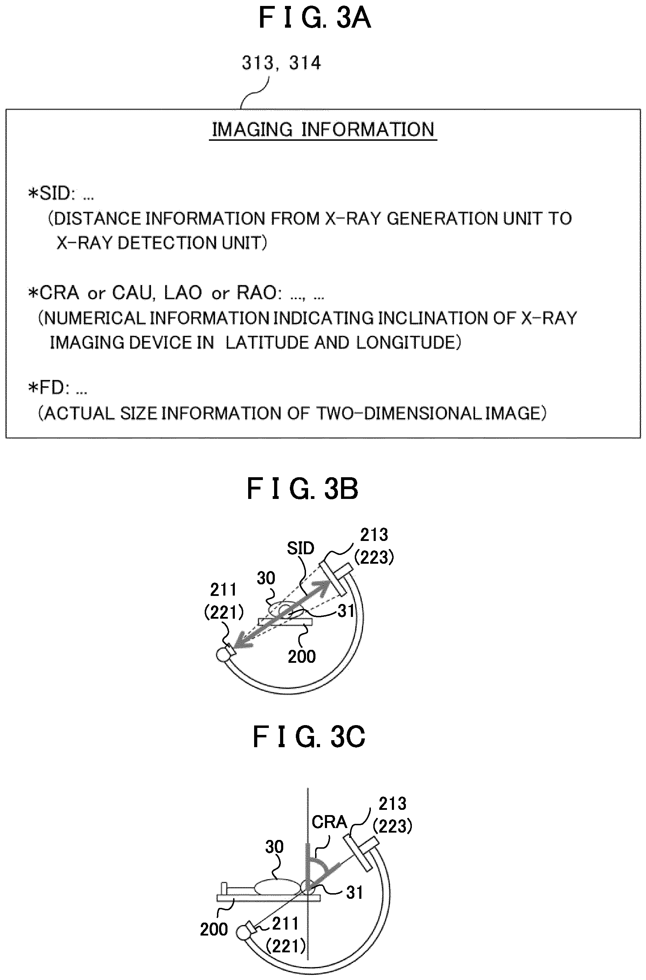

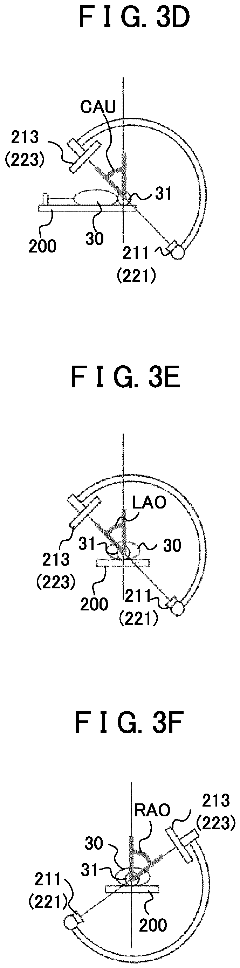

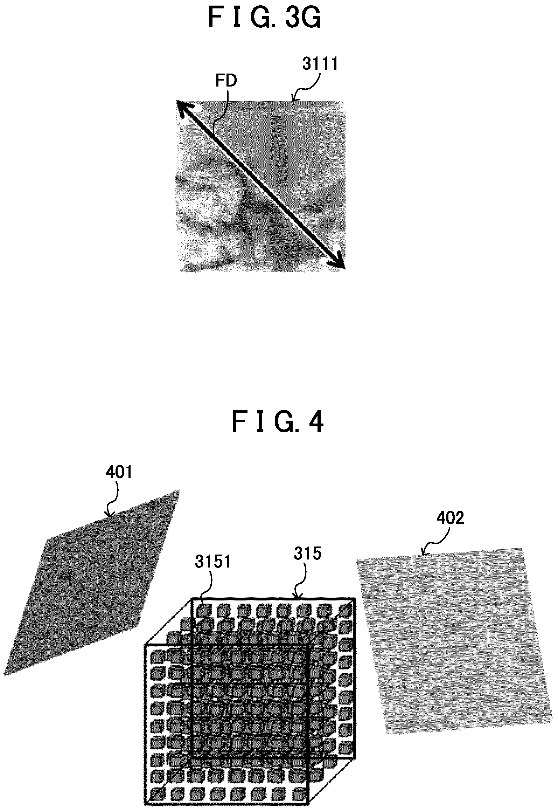

Further, the image/information acquisition unit 310 acquires the first-plane imaging information 313 and the second-plane imaging information 314 from the control device 400 that controls the operations of the first X-ray imaging apparatus 210 and the second X-ray imaging apparatus 220. The first-plane imaging information 313 is imaging information related to X-ray fluoroscopy in the direction of the first plane, and is imaging information for capturing the first-plane X-ray fluoroscopic image 3111. The second-plane imaging information 314 is imaging information related to X-ray fluoroscopy in the direction of the second plane, and is imaging information for capturing the second-plane X-ray fluoroscopic image 3121. Fig. 3A illustrates the embodiment of the present invention and illustrates specific examples of the first-plane imaging information 313 and the second-plane imaging information 314 in Fig. 1. In the present embodiment, the first-plane imaging information 313 and the second-plane imaging information 314 include, as illustrated in Fig. 3A, distance information from the X-ray generation unit to the X-ray detection unit (SID: Source Image Receptor Distance), numerical information indicating the inclination of the X-ray imaging apparatus in latitude and longitude (CRA: Cranial direction (the upper side of the patient 30) or CAU: Caudal direction (the lower side of the patient 30), LAO: Left Anterior Oblique view (the left side of the patient 30) or RAO: Right Anterior Oblique view (the right side of the patient 30)), and actual size information of the two-dimensional image (FD: Flat Detector). Figs. 3B to 3G are diagrams for explaining various types of information included in the first-plane imaging information 313 and the second-plane imaging information 314 illustrated in Fig. 3A, and the same reference numerals are denoted for components that are similar to the components illustrated in Figs. 1 and 2A to 2B. As illustrated in Fig. 3B, the distance information (SID) of the first-plane imaging information 313 is distance information from the X-ray generation unit 211 to the X-ray detection unit 213, and the distance information (SID) of the second-plane imaging information 314 is distance information from the X-ray generation unit 221 to the X-ray detection unit 223. The numerical information (CRA or CAU, LAO or RAO) of the first-plane imaging information 313 includes, as illustrated in Figs. 3C and 3D, CRA or CAU indicating an angle between a vertical line passing through the head 31 of the patient 30 and a line connecting the X-ray generation unit 211 to the X-ray detection unit 213 when the patient 30 is lying on the tabletop 200 and positioned in the horizontal direction, and as illustrated in Figs. 3E and 3F, LAO or RAO indicating an angle between a vertical line passing through the head 31 of the patient 30 and a line connecting the X-ray generation unit 211 to the X-ray detection unit 213 when the patient 30 is lying on the tabletop 200 and positioned in the depth direction. Further, the numerical information (CRA or CAU, LAO or RAO) of the second-plane imaging information 314 includes, as illustrated in Figs. 3C and 3D, CRA or CAU indicating an angle between a vertical line passing through the head 31 of the patient 30 and a line connecting the X-ray generation unit 221 to the X-ray detection unit 223 when the patient 30 is lying on the tabletop 200 and positioned in the horizontal direction, and as illustrated in Figs. 3E and 3F, LAO or RAO indicating an angle between a vertical line passing through the head 31 of the patient 30 and a line connecting the X-ray generation unit 221 to the X-ray detection unit 223 when the patient 30 is lying on the tabletop 200 and positioned in the depth direction. The actual size information (FD) of the two-dimensional image of the first-plane imaging information 313 is information corresponding to the diagonal length of the first-plane X-ray fluoroscopic image 3111 as illustrated in Fig. 3G, and the actual size information (FD) of the two-dimensional image of the second-plane imaging information 314 is information corresponding to the diagonal length of the second-plane X-ray fluoroscopic image 3121. Note that, in the present embodiment, the image/information acquisition unit 310 acquires the first-plane imaging information 313 and the second-plane imaging information 314 from the control device 400 that controls the operations of the first X-ray imaging apparatus 210 and the second X-ray imaging apparatus 220, but the present invention is not limited to this form. For example, a form can also be applied to the present invention, in which the image/information acquisition unit 310 acquires the first-plane imaging information 313 and the second-plane imaging information 314 by performing image analysis on the acquired first-plane roadmap image 3112 and second-plane roadmap image 3122, respectively, and if there is an error in the imaging information thus acquired, the image/information acquisition unit 310 acquires correct imaging information from the input device 500.

Now, return to Fig. 1 again for explanation. Furthermore, the image/information acquisition unit 310 acquires subject lattice point cloud information 315 input from the input device 500 via the control device 400. Fig. 4 illustrates the embodiment of the present invention and is a diagram illustrating a specific example of the subject lattice point cloud information 315 in Fig. 1. Illustrated in Fig. 4 is the subject lattice point cloud information 315 that is information on a plurality of lattice points 3151 set within a rectangular parallelepiped that is specified as a rectangular parallelepiped region in the head 31 of the patient 30, who is a subject. Further, illustrated in Fig. 4 are also a first plane 401 that is a plane between the X-ray generation unit 211 and the X-ray detection unit 213 in the first X-ray imaging apparatus 210 and is parallel to the X-ray detection surface 213a of the X-ray detection unit 213, and a second plane 402 that is a plane between the X-ray generation unit 221 and the X-ray detection unit 223 in the second X-ray imaging apparatus 220 and is parallel to the X-ray detection surface 223a of the X-ray detection unit 223. In this case, the X-ray detection surface 213a of the X-ray detection unit 213 is positioned in the direction of the first plane relative to the head 31 of the patient 30, who is a subject, and the X-ray detection surface 223a of the X-ray detection unit 223 is positioned in the direction of the second plane relative to the head 31 of the patient 30, who is a subject.

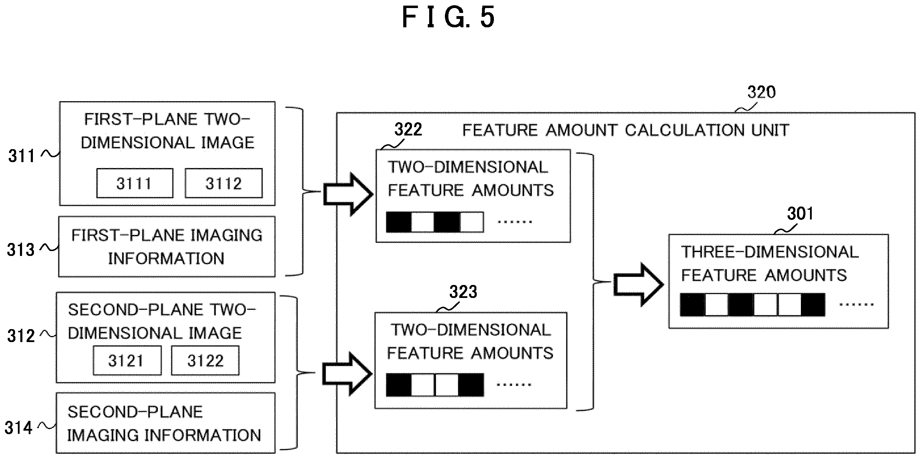

Now, return to Fig. 1 again for explanation. The feature amount calculation unit 320 calculates three-dimensional feature amounts 301 by machine learning. Specifically, the feature amount calculation unit 320 calculates three-dimensional feature amounts 301 of the skull and blood vessel of the head 31 of the patient 30 and the medical device based on the first-plane two-dimensional image 311, the second-plane two-dimensional image 312, the first-plane imaging information 313, and the second-plane imaging information 314, which are acquired by the image/information acquisition unit 310. In the present embodiment, the feature amount calculation unit 320 calculates the three-dimensional feature amounts 301 of the skull and blood vessel of the head 31 of the patient 30 and the medical device by using a first learned model 321 that has been machine-learned to output three-dimensional feature amounts of the skull, blood vessel, and medical device in response to inputs of the first-plane two-dimensional image 311, the second-plane two-dimensional image 312, the first-plane imaging information 313, and the second-plane imaging information 314. In this case, machine learning with the first learned model 321 to be performed is machine learning, for example, using a neural network model (NN model). Fig. 5 illustrates the embodiment of the present invention and is a diagram illustrating an example of processing performed by the feature amount calculation unit 320 in Fig. 1. In Fig. 5, the same components as those illustrated in Fig. 1 are denoted by the same reference numerals. As illustrated in Fig. 5, the feature amount calculation unit 320 first calculates two-dimensional feature amounts 322 of the skull, blood vessel, and medical device by using the first-plane two-dimensional image 311 and the first-plane imaging information 313, and then calculates two-dimensional feature amounts 323 of the skull, blood vessel, and medical device by using the second-plane two-dimensional image 312 and the second-plane imaging information 314. Then, the feature amount calculation unit 320 calculates three-dimensional feature amounts 301 of the skull and blood vessel of the head 31 of the patient 30, and the medical device by machine learning using NeRF (Neural Radiance Fields), which generates a free viewpoint image, from the two-dimensional feature amounts 322 for the first plane and the two-dimensional feature amounts 323 for the second plane.

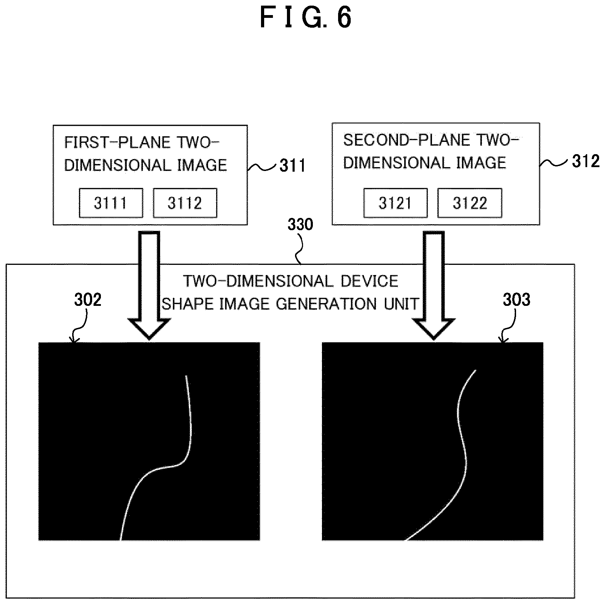

Now, return to Fig. 1 again for explanation. The two-dimensional device shape image generation unit 330 generates a first-plane two-dimensional device shape image 302 representing a shape of the medical device 100 in the direction of the first plane based on the first-plane two-dimensional image 311 acquired by the image/information acquisition unit 310, and generates a second-plane two-dimensional device shape image 303 representing a shape of the medical device 100 in the direction of the second plane based on the second-plane two-dimensional image 312 acquired by the image/information acquisition unit 310. In the present embodiment, the two-dimensional device shape image generation unit 330 generates the first-plane two-dimensional device shape image 302 and the second-plane two-dimensional device shape image 303 by using a second learned model 331 that has been machine-learned to output a first-plane two-dimensional device shape image representing a shape of the medical device 100 in the direction of the first plane and a second-plane two-dimensional device shape image representing a shape of the medical device 100 in the direction of the second plane in response to inputs of the first-plane two-dimensional image 311 and the second-plane two-dimensional image 312. In this case, machine learning with the second learned model 331 to be performed is machine learning, for example, using an NN model. Fig. 6 illustrates the embodiment of the present invention and is a diagram illustrating an example of processing performed by the two-dimensional device shape image generation unit 330 in Fig. 1. In Fig. 6, the same components as those illustrated in Fig. 1 are denoted by the same reference numerals. As illustrated in Fig. 6, the two-dimensional device shape image generation unit 330 generates the first-plane two-dimensional device shape image 302 by segmenting the first-plane two-dimensional image 311 with an NN model (color-coding the image for each object), and also generates the second-plane two-dimensional device shape image 303 by segmenting the second-plane two-dimensional image 312 with an NN model. Note that, in the first-plane two-dimensional device shape image 302 and the second-plane two-dimensional device shape image 303 illustrated in Fig. 6, a portion corresponding to the medical device 100 is depicted in white.

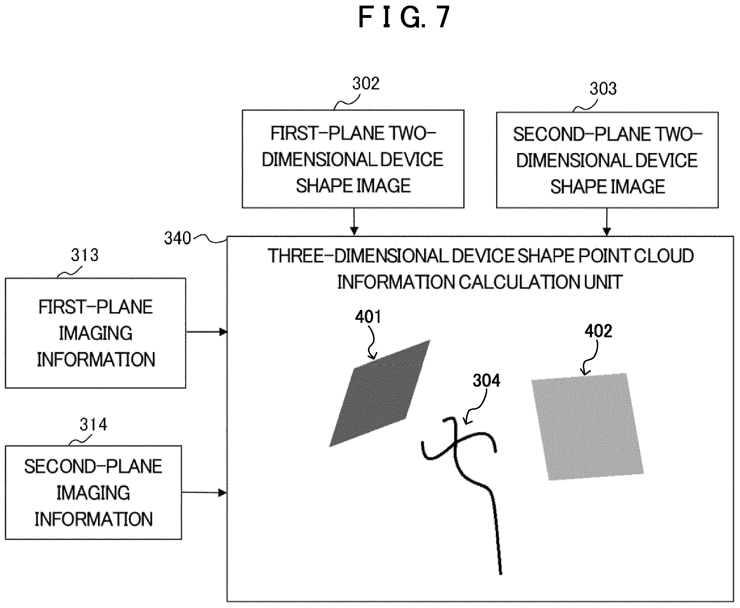

Now, return to Fig. 1 again for explanation. The three-dimensional device shape point cloud information calculation unit 340 calculates three-dimensional device shape point cloud information 304 that is point cloud information of the three-dimensional shape of the medical device 100, based on the first-plane two-dimensional device shape image 302 and the second-plane two-dimensional device shape image 303 which are generated by the two-dimensional device shape image generation unit 330, and based on the first-plane imaging information 313 and the second-plane imaging information 314 which are acquired by the image/information acquisition unit 310. Fig. 7 illustrates the embodiment of the present invention and is a diagram illustrating an example of processing performed by the three-dimensional device shape point cloud information calculation unit 340 in Fig. 1. In Fig. 7, the same components as those illustrated in Fig. 1 are denoted by the same reference numerals. As illustrated in Fig. 7, the three-dimensional device shape point cloud information calculation unit 340 calculates three-dimensional device shape point cloud information 304 based on the first-plane two-dimensional device shape image 302, the second-plane two-dimensional device shape image 303, the first-plane imaging information 313, and the second-plane imaging information 314. In this case, the three-dimensional device shape point cloud information 304 illustrated in Fig. 7 may include not only a real image of the medical device 100 but also a virtual image. Further, Fig. 7 also illustrates a first plane 401 and a second plane 402 defined in the same manner as in Fig. 4.

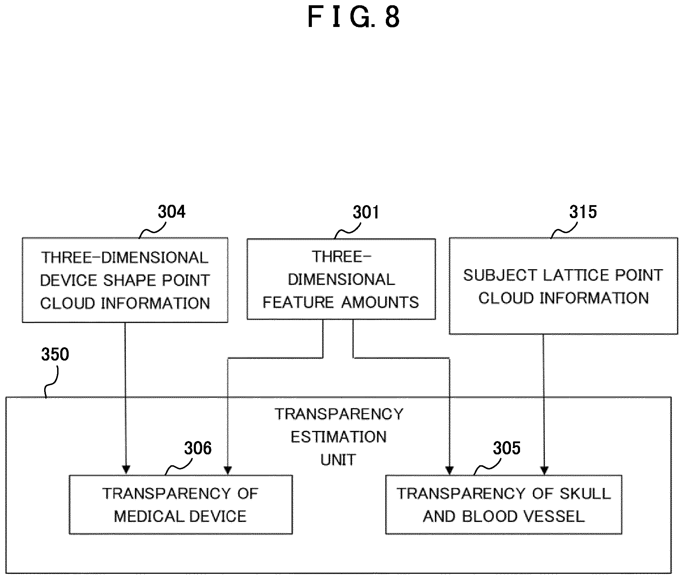

Now, return to Fig. 1 again for explanation. The transparency estimation unit 350 estimates a transparency 305 of the skull and blood vessel based on the three-dimensional feature amounts 301 of the skull, blood vessel, and medical device calculated by the feature amount calculation unit 320 and the subject lattice point cloud information 315 acquired by the image/information acquisition unit 310, and estimates a transparency 306 of the medical device 100 based on the three-dimensional feature amounts 301 of the skull, blood vessel, and medical device calculated by the feature amount calculation unit 320 and the three-dimensional device shape point cloud information 304 calculated by the three-dimensional device shape point cloud information calculation unit 340. In the present embodiment, the transparency estimation unit 350 estimates the transparency 305 of the skull and blood vessel and the transparency 306 of the medical device 100 by using a third learned model 351 that has been machine-learned to output a transparency of the skull and blood vessel in response to inputs of the three-dimensional feature amounts 301 of the skull, blood vessel, and medical device and the subject lattice point cloud information 315, and that has been machine-learned to output a transparency of the medical device 100 in response to inputs of the three-dimensional feature amounts 301 of the skull, blood vessel, and medical device and the three-dimensional device shape point cloud information 304. In this case, machine learning with the third learned model 351 to be performed is machine learning, for example, using an NN model. Fig. 8 illustrates the embodiment of the present invention and is a diagram illustrating an example of processing performed by the transparency estimation unit 350 in Fig. 1. In Fig. 8, the same components as those illustrated in Fig. 1 are denoted by the same reference numerals. As illustrated in Fig. 8, the transparency estimation unit 350 estimates the transparency (the degree to which the image is transparent and allows the color behind it to be seen) 305 of the skull and blood vessel at each of the plurality of lattice points 3151 in the subject lattice point cloud information 315 by using an NN model, on the basis of the three-dimensional feature amounts 301 of the skull, blood vessel, and medical device calculated by the feature amount calculation unit 320. Further, as illustrated in Fig. 8, the transparency estimation unit 350 estimates the transparency 306 of the medical device 100 on the point cloud in the three-dimensional device shape point cloud information 304 by using an NN model on the basis of the three-dimensional feature amounts 301 of the skull, blood vessel, and medical device calculated by the feature amount calculation unit 320.

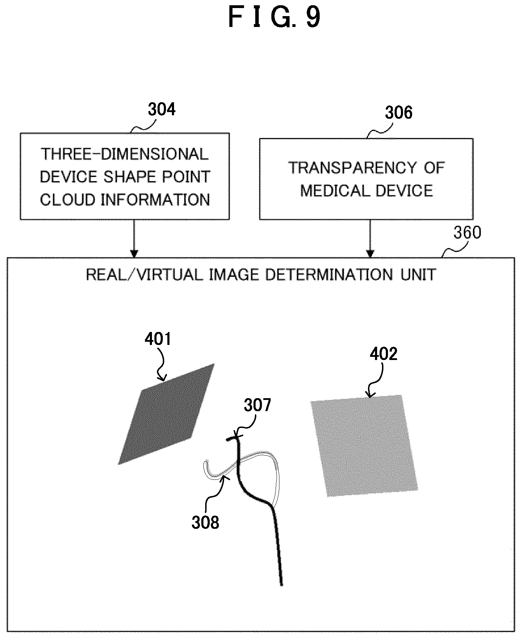

Now, return to Fig. 1 again for explanation. The real/virtual image determination unit 360 determines, based on the transparency 306 of the medical device 100 estimated by the transparency estimation unit 350, whether the three-dimensional device shape point cloud information 304 indicates a real image or a virtual image for each of a plurality of groups into which a nearby region including the point cloud in the three-dimensional device shape point cloud information 304 is divided. Specifically, the real/virtual image determination unit 360 calculates an average value of the transparency 306 of the medical device 100 for each group, determines that a group for which the average value is equal to or less than a predetermined threshold value is a real image, and determines that a group for which the average value is greater than the predetermined threshold value is a virtual image. Here, the reason why the determination as to whether the three-dimensional device shape point cloud information 304 indicates a real image or a virtual image is made by taking into consideration not only the point cloud in the three-dimensional device shape point cloud information 304 but also the nearby region including the point cloud in the three-dimensional device shape point cloud information 304 is to reduce the determination error. Further, the reason why the group for which the average value of the transparency 306 of the medical device 100 is equal to or less than the predetermined threshold value is determined to be a real image is that the medical device 100 is displayed with a small transparency in an X-ray fluoroscopic image or the like. Fig. 9 illustrates the embodiment of the present invention and is a diagram illustrating an example of processing performed by the real/virtual image determination unit 360 in Fig. 1. In Fig. 9, the same components as those illustrated in Figs. 1 and 7 are denoted by the same reference numerals. As illustrated in Fig. 9, the real/virtual image determination unit 360 determines, on the basis of the transparency 306 of the medical device 100 estimated by the transparency estimation unit 350, whether the three-dimensional device shape point cloud information 304 indicates a real image or a virtual image for each of a plurality of groups into which a nearby region including the point cloud in the three-dimensional device shape point cloud information 304 is divided. Specifically, Fig. 9 illustrates an example of an image of a three-dimensional device shape point cloud information 307 of a point cloud belonging to a group determined to be a real image, and an image of a three-dimensional device shape point cloud information 308 of a point cloud belonging to a group determined to be a virtual image, in the three-dimensional device shape point cloud information 304 illustrated in Fig. 7.

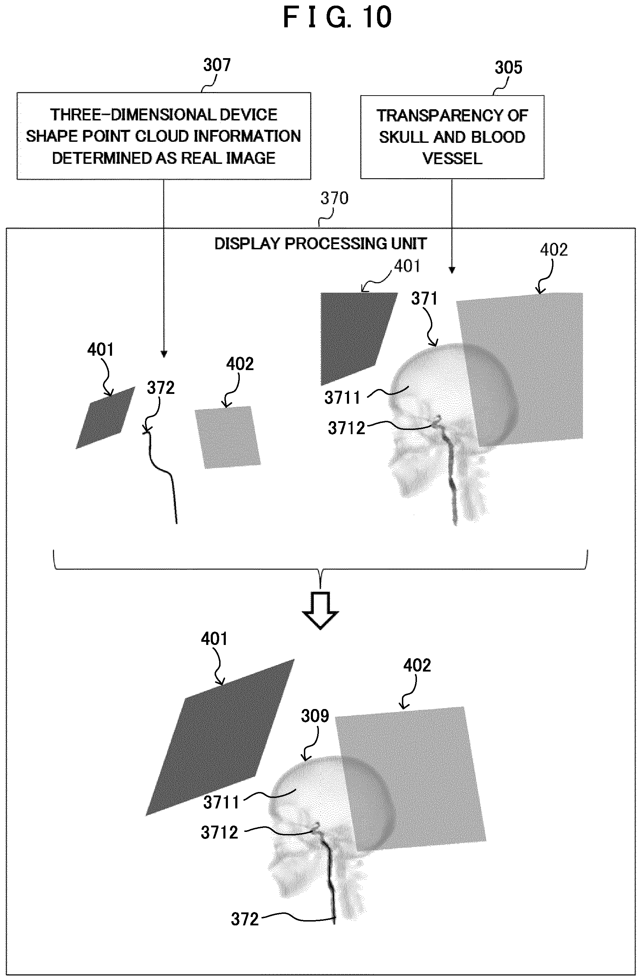

Now, return to Fig. 1 again for explanation. The display processing unit 370 generates three-dimensional skull and three-dimensional blood vessel images 371 based on the transparency 305 of the skull and blood vessel estimated by the transparency estimation unit 350, and generates a three-dimensional medical device image 372 based on the three-dimensional device shape point cloud information 307 of the point cloud belonging to the group determined to be a real image by the real/virtual image determination unit 360. Then, the display processing unit 370 generates a three-dimensional medical image 309 by superimposing the three-dimensional skull and three-dimensional blood vessel images 371 and the three-dimensional medical device image 372, and performs processing of displaying the generated three-dimensional medical image 309 on the display unit 380. Fig. 10 illustrates the embodiment of the present invention and is a diagram illustrating an example of processing performed by the display processing unit 370 in Fig. 1. In Fig. 10, the same components as those illustrated in Figs. 1, 4, 8, and 9 are denoted by the same reference numerals. As illustrated in Fig. 10, the display processing unit 370 generates, by using the transparency 305 of the skull and blood vessel estimated by the transparency estimation unit 350, the three-dimensional skull and three-dimensional blood vessel images 371, for example, by volume rendering. In this case, the three-dimensional skull and three-dimensional blood vessel images 371 illustrated in Fig. 10 include a three-dimensional skull image 3711 and a three-dimensional blood vessel image 3712. Further, as illustrated in Fig. 10, the display processing unit 370 generates the three-dimensional medical device image 372 by using the three-dimensional device shape point cloud information 307 of the point cloud belonging to the group determined to be a real image by the real/virtual image determination unit 360. Furthermore, as illustrated in Fig. 10, the display processing unit 370 generates the three-dimensional medical image 309 by superimposing the three-dimensional skull and three-dimensional blood vessel images 371 and the three-dimensional medical device image 372. Then, the display processing unit 370 performs processing of displaying, on the display unit 380, the generated three-dimensional medical image 309 illustrated in Fig. 10.

Now, return to Fig. 1 again for explanation. The display unit 380 is a display unit for the surgeon 20 to view an operation on a blood vessel in the head 31 of the patient 30, who is a subject, using the medical device 100. The display unit 380 displays various types of images and various types of information under the control of the control device 400. In the present embodiment, the display unit 380 displays the three-dimensional medical image 309 generated by the display processing unit 370.

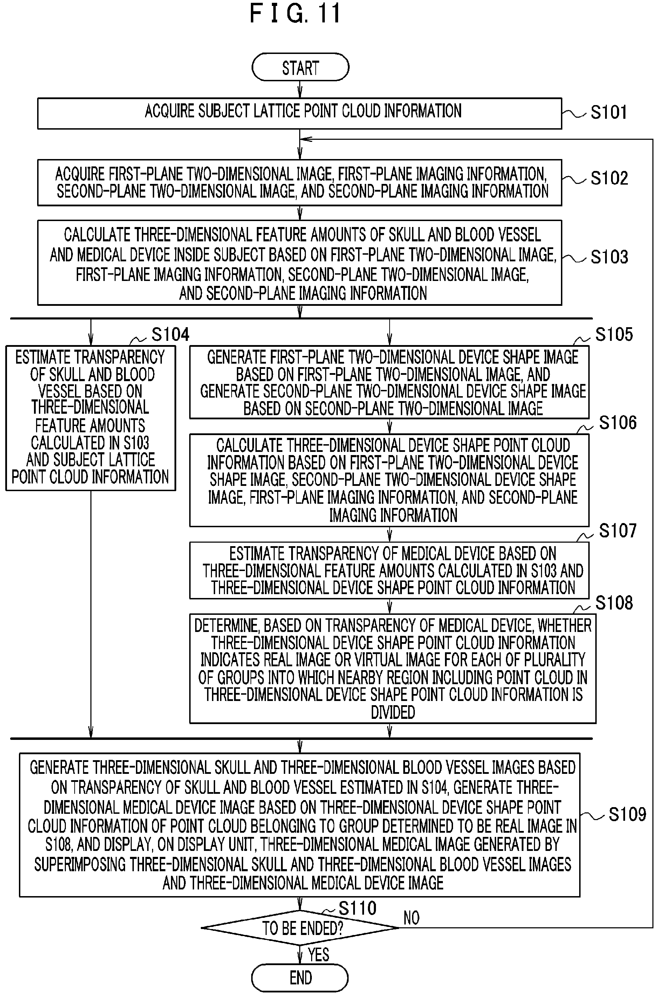

Next, a procedure of a medical image processing method using the medical image processing apparatus 300 in Fig. 1 will be described. Fig. 11 is a flowchart illustrating an example of a processing procedure of a medical image processing method by the medical image processing apparatus 300 according to the embodiment of the present invention.

First, in step S101 of Fig. 11, the image/information acquisition unit 310 acquires the subject lattice point cloud information 315 input from the input device 500 via the control device 400.

Next, in step S102, the image/information acquisition unit 310 acquires the first-plane two-dimensional image 311 from the X-ray detection unit 213 of the first X-ray imaging apparatus 210, acquires the second-plane two-dimensional image 312 from the X-ray detection unit 223 of the second X-ray imaging apparatus 220, and further acquires the first-plane imaging information 313 and the second-plane imaging information 314 from the control device 400.

Next, in step S103, the feature amount calculation unit 320 calculates the three-dimensional feature amounts 301 of the skull and blood vessel of the head 31 of the patient 30, and the medical device based on the first-plane two-dimensional image 311, the second-plane two-dimensional image 312, the first-plane imaging information 313, and the second-plane imaging information 314, which are acquired in step S102.

Next, in step S104, the transparency 305 of the skull and blood vessel is estimated based on the three-dimensional feature amounts 301 of the skull, blood vessel, and medical device calculated in step S103 and the subject lattice point cloud information 315 acquired in step S101.

In the present embodiment, the following processes of steps S105 to S108 are performed in parallel with the process of step S104.

In step S105, the two-dimensional device shape image generation unit 330 generates the first-plane two-dimensional device shape image 302 based on the first-plane two-dimensional image 311 acquired in step S102, and generates the second-plane two-dimensional device shape image 303 based on the second-plane two-dimensional image 312 acquired in step S102.

Next, in step S106, the three-dimensional device shape point cloud information calculation unit 340 calculates the three-dimensional device shape point cloud information 304, which is point cloud information of a three-dimensional shape of the medical device 100, based on the first-plane two-dimensional device shape image 302 and the second-plane two-dimensional device shape image 303 generated in step S105, and the first-plane imaging information 313 and the second-plane imaging information 314 acquired in step S102.

Next, in step S107, the transparency estimation unit 350 estimates the transparency 306 of the medical device 100 based on the three-dimensional feature amounts 301 of the skull, blood vessel, and medical device calculated in step S103 and the three-dimensional device shape point cloud information 304 calculated in step S106.

Next, in step S108, the real/virtual image determination unit 360 determines, based on the transparency 306 of the medical device 100 estimated in step S107, whether the three-dimensional device shape point cloud information 304 indicates a real image or a virtual image for each of a plurality of groups into which a nearby region including the point cloud in the three-dimensional device shape point cloud information 304 is divided.

When the processes of steps S104 and S108 ends, the processing proceeds to step S109. At step S109 to which the processing proceeds, the display processing unit 370 first generates the three-dimensional skull and three-dimensional blood vessel images 371 based on the transparency 305 of the skull and blood vessel estimated in step S104, and generates the three-dimensional medical device image 372 based on the three-dimensional device shape point cloud information 307 of the point cloud belonging to the group determined to be a real image in step S108. Next, the display processing unit 370 generates the three-dimensional medical image 309 by superimposing the three-dimensional skull and three-dimensional blood vessel images 371 and the three-dimensional medical device image 372, and performs processing of displaying the generated three-dimensional medical image 309 on the display unit 380.

Next, in step S110, the medical image processing apparatus 300 (e.g., the image/information acquisition unit 310) determines, based on information input from the input device 500 via the control device 400, whether or not to end the vascular operation on the subject (in the present embodiment, the head 31 of the patient 30) using the medical device 100.

If it is determined in step S110 that the operation on the blood vessel of the subject using the medical device 100 is not to be ended (S110/NO), the processing returns to step S102 to perform the processes of step S102 and the subsequent steps again.

On the other hand, if the result of the determination in step S110 is that the operation on the blood vessel of the subject using the medical device 100 is to be ended (S110/YES), the processing of the flowchart illustrated in Fig. 11 is ended.

The medical image processing apparatus 300 according to the embodiment of the present invention described above performs the following processing. The feature amount calculation unit 320 calculates the three-dimensional feature amounts 301 of the skull and blood vessel of the subject (the head 31 of the patient 30) and the medical device 100 based on the first-plane two-dimensional image 311, the first-plane imaging information 313, the second-plane two-dimensional image 312, and the second-plane imaging information 314. Further, the two-dimensional device shape image generation unit 330 generates the first-plane two-dimensional device shape image 302 based on the first-plane two-dimensional image 311, and generates the second-plane two-dimensional device shape image 303 based on the second-plane two-dimensional image 312. The three-dimensional device shape point cloud information calculation unit 340 calculates the three-dimensional device shape point cloud information 304 based on the first-plane two-dimensional device shape image 302, the second-plane two-dimensional device shape image 303, the first-plane imaging information 313, and the second-plane imaging information 314. The transparency estimation unit 350 estimates the transparency 305 of the skull and blood vessel based on the three-dimensional feature amounts 301 calculated by the feature amount calculation unit 320 and the subject lattice point cloud information 315, and estimates the transparency 306 of the medical device 100 based on the three-dimensional feature amounts 301 calculated by the feature amount calculation unit 320 and the three-dimensional device shape point cloud information 304. The real/virtual image determination unit 360 determines, based on the transparency 306 of the medical device 100 estimated by the transparency estimation unit 350, whether the three-dimensional device shape point cloud information 304 indicates a real image or a virtual image for each of a plurality of groups into which a nearby region including the point cloud in the three-dimensional device shape point cloud information 304 is divided. Then, the display processing unit 370 generates three-dimensional skull and three-dimensional blood vessel images 371 based on the transparency 305 of the skull and blood vessel estimated by the transparency estimation unit 350, generates a three-dimensional medical device image 372 based on the three-dimensional device shape point cloud information 307 of the point cloud belonging to the group determined to be a real image by the real/virtual image determination unit 360, and performs processing of displaying, on the display unit 380, the three-dimensional medical image 309 generated by superimposing the three-dimensional skull and three-dimensional blood vessel images 371 and the three-dimensional medical device image 372.
With this configuration, for a surgeon 20 to perform an operation on a blood vessel of a subject using the medical device 100 while viewing a medical image, it is possible to more easily recognize a three-dimensional positional relationship between the skull, the blood vessel, and the medical device, thereby performing the operation safely.

Note that the inventor(s) of the present application have found that the estimation accuracy of the transparency estimation unit 350 is poor in estimating the transparency of the medical device 100 based on the three-dimensional feature amounts 301 calculated by the feature amount calculation unit 320 and the subject lattice point cloud information 315. Thus, in the medical image processing apparatus 300 according to the embodiment of the present invention, with regard to the medical device 100, the transparency estimation unit 350 estimates the transparency 306 of the medical device 100 based on the three-dimensional feature amounts 301 calculated by the feature amount calculation unit 320, as well as the three-dimensional device shape point cloud information 304 obtained as a result of the processing of the two-dimensional device shape image generation unit 330 and the three-dimensional device shape point cloud information calculation unit 340; further, the real/virtual image determination unit 360 determines, based on the transparency 306 of the medical device 100, whether the three-dimensional device shape point cloud information 304 indicates a real image or a virtual image for each of a plurality of groups into which a nearby region including the point cloud in the three-dimensional device shape point cloud information 304 is divided; and further, the display processing unit 370 generates the three-dimensional medical device image 372 based on the three-dimensional device shape point cloud information 307 of the point cloud belonging to the group determined to be a real image by the real/virtual image determination unit 360.

### (Other Embodiments)

In the above-described embodiment of the present invention, an example has been described in which the head 31 of the patient 30 is used as a subject to be subjected to X-ray fluoroscopy. However, the present invention is not limited to this. For example, the present invention can also be applied to a form in which the chest or abdomen of the patient 30 is used as a subject to be subjected to X-ray fluoroscopy. In the other embodiment in which the chest or abdomen of the patient 30 is used as a subject to be subjected to X-ray fluoroscopy, the medical device 100 is, for example, a stent graft instrument for performing stent-graft placement on an aneurysm of the thoracic aorta inside the chest of the patient 30 or an aneurysm of the abdominal aorta inside the abdomen of the patient 30. Further, in the other embodiment in which the chest or abdomen of the patient 30 is used as a subject to be subjected to X-ray fluoroscopy, the medical image processing apparatus 300 performs processing related only to a "blood vessel" assuming the thoracic aorta or abdominal aorta, instead of the processing related to the "skull" and "blood vessel" in the above-described embodiment of the present invention. Specifically, in the case of the other embodiment in which the chest or abdomen of the patient 30 is used as a subject to be subjected to X-ray fluoroscopy, the medical image processing apparatus 300 performs the following processing.
The feature amount calculation unit 320 calculates three-dimensional feature amounts 301 of the blood vessel and the medical device 100 based on a first-plane two-dimensional image 311 of the subject (the chest or abdomen of the patient 30) including the blood vessel (thoracic aorta or abdominal aorta) into which the medical device 100 is inserted, captured by X-ray fluoroscopy in the direction of the first plane, and first-plane imaging information 313, and a second-plane two-dimensional image 312 of the subject captured by X-ray fluoroscopy in the direction of the second plane different from the first plane, and second-plane imaging information 314. The two-dimensional device shape image generation unit 330 generates a first-plane two-dimensional device shape image 302 based on the first-plane two-dimensional image 311, and generates a second-plane two-dimensional device shape image 303 based on the second-plane two-dimensional image 312. The three-dimensional device shape point cloud information calculation unit 340 calculates three-dimensional device shape point cloud information 304, which is point cloud information of a three-dimensional shape of the medical device 100, based on the first-plane two-dimensional device shape image 302, the second-plane two-dimensional device shape image 303, the first-plane imaging information 313, and the second-plane imaging information 314. The transparency estimation unit 350 estimates a transparency 305 of the blood vessel based on the three-dimensional feature amounts 301 calculated by the feature amount calculation unit 320 and subject lattice point cloud information 315, and estimates a transparency 306 of the medical device 100 based on the three-dimensional feature amounts 301 calculated by the feature amount calculation unit 320 and the three-dimensional device shape point cloud information 304. The real/virtual image determination unit 360 determines, based on the transparency 306 of the medical device 100 estimated by the transparency estimation unit 350, whether the three-dimensional device shape point cloud information 304 indicates a real image or a virtual image for each of a plurality of groups into which a nearby region including the point cloud in the three-dimensional device shape point cloud information 304 is divided. The display processing unit 370 generates a three-dimensional blood vessel image 371 based on the transparency 305 of the blood vessel estimated by the transparency estimation unit 350, generates a three-dimensional medical device image 372 based on the three-dimensional device shape point cloud information 307 of a point cloud belonging to the group determined to be a real image by the real/virtual image determination unit 360, and performs processing of displaying, on the display unit 380, a three-dimensional medical image 309 generated by superimposing the three-dimensional blood vessel image 371 and the three-dimensional medical device image 372.
With this configuration, for a surgeon 20 to perform an operation on a blood vessel of a subject using the medical device 100 while viewing a medical image, it is possible to more easily recognize a three-dimensional positional relationship between the blood vessel and the medical device, thereby performing the operation safely.

The present invention may also be achieved by processing of supplying a program that implements one or more functions of the above-described embodiments to a system or apparatus via a network or a storage medium, and reading and executing the program by one or more processors in a computer of the system or apparatus. It may also be achieved by a circuit (e.g., ASIC) that implements the one or more functions.
This program and a computer-readable storage medium having the program stored are included in the present invention.

Note that the above-described embodiments of the present invention are merely illustrative examples of how the present invention may be implemented, and the technical scope of the present invention should not be construed as being limited thereby. That is, the present invention can be implemented in various forms without departing from its technical concept or main features.

## Claims

1. A medical image processing apparatus including a display unit for a surgeon to view an operation on a blood vessel of a subject using a medical device, the medical image processing apparatus comprising:
a feature amount calculation unit configured to calculate three-dimensional feature amounts of the blood vessel and the medical device based on a first-plane two-dimensional image, first-plane imaging information, a second-plane two-dimensional image, and second-plane imaging information, the first-plane two-dimensional image including a first-plane X-ray fluoroscopic image of the subject including the blood vessel into which the medical device is inserted, captured in a direction of a first-plane by X-ray fluoroscopy, and including a first-plane roadmap image corresponding to the first-plane X-ray fluoroscopic image, the first-plane imaging information being related to X-ray fluoroscopy in the direction of the first plane, the second-plane two-dimensional image including a second-plane X-ray fluoroscopic image of the subject including the blood vessel into which the medical device is inserted, captured in a direction of a second-plane different from the direction of the first plane by X-ray fluoroscopy, and including a second-plane roadmap image corresponding to the second-plane X-ray fluoroscopic image, the second-plane imaging information being related to X-ray fluoroscopy in the direction of the second plane;
a two-dimensional device shape image generation unit configured to generate a first-plane two-dimensional device shape image representing a shape of the medical device in the direction of the first plane based on the first-plane two-dimensional image, and generate a second-plane two-dimensional device shape image representing a shape of the medical device in the direction of the second plane based on the second-plane two-dimensional image;
a three-dimensional device shape point cloud information calculation unit configured to calculate three-dimensional device shape point cloud information that is point cloud information of a three-dimensional shape of the medical device, based on the first-plane two-dimensional device shape image, the second-plane two-dimensional device shape image, the first-plane imaging information, and the second-plane imaging information;
a transparency estimation unit configured to estimate a transparency of the blood vessel based on the three-dimensional feature amounts calculated by the feature amount calculation unit and subject lattice point cloud information that is information on a plurality of lattice points set for the subject, and estimate a transparency of the medical device based on the three-dimensional feature amounts calculated by the feature amount calculation unit and the three-dimensional device shape point cloud information;
a real/virtual image determination unit configured to determine, based on the transparency of the medical device estimated by the transparency estimation unit, whether the three-dimensional device shape point cloud information indicates a real image or a virtual image for each of a plurality of groups into which a nearby region including a point cloud in the three-dimensional device shape point cloud information is divided; and
a display processing unit configured to generate a three-dimensional blood vessel image based on the transparency of the blood vessel estimated by the transparency estimation unit, generate a three-dimensional medical device image based on the three-dimensional device shape point cloud information of the point cloud belonging to the group determined to be a real image by the real/virtual image determination unit, and perform processing of displaying on the display unit a three-dimensional medical image generated by superimposing the three-dimensional blood vessel image and the three-dimensional medical device image.

2. The medical image processing apparatus according to claim 1, wherein the real/virtual image determination unit calculates an average value of the transparency of the medical device for each group, determines that a group for which the average value is equal to or less than a predetermined threshold value is a real image, and determines that a group for which the average value is greater than the predetermined threshold value is a virtual image.

3. The medical image processing apparatus according to claim 1, wherein
the subject is a head of a patient,
the first-plane X-ray fluoroscopic image is an image of the blood vessel of the head and a skull of the head into which the medical device is inserted, captured in the direction of the first plane by X-ray fluoroscopy,
the second-plane X-ray fluoroscopic image is an image of the blood vessel and the skull of the head into which the medical device is inserted, captured in the direction of the second plane by X-ray fluoroscopy,
the feature amount calculation unit calculates three-dimensional feature amounts of the skull, the blood vessel, and the medical device,
the transparency estimation unit estimates a transparency of the skull and the blood vessel based on the three-dimensional feature amounts calculated by the feature amount calculation unit and the subject lattice point cloud information, and
the display processing unit generates a three-dimensional skull image and a three-dimensional blood vessel image based on the transparency of the skull and the blood vessel estimated by the transparency estimation unit, and performs processing of displaying, on the display unit, a three-dimensional medical image generated by superimposing the three-dimensional skull and three-dimensional blood vessel images and the three-dimensional medical device image.

4. The medical image processing apparatus according to claim 1, wherein the subject is a chest or an abdomen of a patient.

5. The medical image processing apparatus according to claim 1, wherein the feature amount calculation unit calculates the three-dimensional feature amounts using a learned model that has been machine-learned to output three-dimensional feature amounts of the blood vessel and the medical device in response to inputs of the first-plane two-dimensional image, the first-plane imaging information, the second-plane two-dimensional image, and the second-plane imaging information.

6. The medical image processing apparatus according to claim 1, wherein the two-dimensional device shape image generation unit generates the first-plane two-dimensional device shape image and the second-plane two-dimensional device shape image using a learned model that has been machine-learned to output the first-plane two-dimensional device shape image and the second-plane two-dimensional device shape image in response to inputs of the first-plane two-dimensional image and the second-plane two-dimensional image.

7. The medical image processing apparatus according to claim 1, wherein the transparency estimation unit estimates the transparency of the blood vessel and the transparency of the medical device using a learned model that has been machine-learned to output a transparency of the blood vessel in response to inputs of the three-dimensional feature amounts calculated by the feature amount calculation unit and the subject lattice point cloud information, and that has been machine-learned to output a transparency of the medical device in response to inputs of the three-dimensional feature amounts calculated by the feature amount calculation unit and the three-dimensional device shape point cloud information.

8. A medical image processing method performed by a medical image processing apparatus including a display unit for a surgeon to view an operation on a blood vessel of a subject using a medical device, the medical image processing method comprising:
a feature amount calculation step of calculating three-dimensional feature amounts of the blood vessel and the medical device based on a first-plane two-dimensional image, first-plane imaging information, a second-plane two-dimensional image, and second-plane imaging information, the first-plane two-dimensional image including a first-plane X-ray fluoroscopic image of the subject including the blood vessel into which the medical device is inserted, captured in a direction of a first-plane by X-ray fluoroscopy, and including a first-plane roadmap image corresponding to the first-plane X-ray fluoroscopic image, the first-plane imaging information being related to X-ray fluoroscopy in the direction of the first plane, the second-plane two-dimensional image including a second-plane X-ray fluoroscopic image of the subject including the blood vessel into which the medical device is inserted, captured in a direction of a second-plane different from the direction of the first plane by X-ray fluoroscopy, and including a second-plane roadmap image corresponding to the second-plane X-ray fluoroscopic image, the second-plane imaging information being related to X-ray fluoroscopy in the direction of the second plane;
a two-dimensional device shape image generation step of generating a first-plane two-dimensional device shape image representing a shape of the medical device in the direction of the first plane based on the first-plane two-dimensional image, and generating a second-plane two-dimensional device shape image representing a shape of the medical device in the direction of the second plane based on the second-plane two-dimensional image;
a three-dimensional device shape point cloud information calculation step of calculating three-dimensional device shape point cloud information that is point cloud information of a three-dimensional shape of the medical device, based on the first-plane two-dimensional device shape image, the second-plane two-dimensional device shape image, the first-plane imaging information, and the second-plane imaging information;
a transparency estimation step of estimating a transparency of the blood vessel based on the three-dimensional feature amounts calculated at the feature amount calculation step and subject lattice point cloud information that is information on a plurality of lattice points set for the subject, and estimating a transparency of the medical device based on the three-dimensional feature amounts calculated at the feature amount calculation step and the three-dimensional device shape point cloud information;
a real/virtual image determination step of determining, based on the transparency of the medical device estimated at the transparency estimation step, whether the three-dimensional device shape point cloud information indicates a real image or a virtual image for each of a plurality of groups into which a nearby region including a point cloud in the three-dimensional device shape point cloud information is divided; and
a display processing step of generating a three-dimensional blood vessel image based on the transparency of the blood vessel estimated at the transparency estimation step, generating a three-dimensional medical device image based on the three-dimensional device shape point cloud information of the point cloud belonging to the group determined to be a real image at the real/virtual image determination step, and performing processing of displaying on the display unit a three-dimensional medical image generated by superimposing the three-dimensional blood vessel image and the three-dimensional medical device image.
